# FASCICULE DE BREVET EUROPEEN

(11) **EP 1 005 857 B1**
(45) Date de publication et mention de la délivrance du brevet: **27.03.2002**
(21) Numéro de dépôt: 99402788.6
(22) Date de dépôt: 09.11.1999
(51) Int. Cl.: A61K 7/48

(54) **Composition sous forme d'émulsion H/E à forte teneur en cire et ses utilisations dans les domaines cosmétiques et dermatologiques**
O/W-EMULSIONEN MIT HOHEM WACHSGEHALT UND DEREN VERWENDUNG IM KOSMETISCHEN UND DERMATOLOGISCHEN BEREICH
O/W-EMULSIONS WITH A HIGH WAX CONTENT AND THEIR USE IN THE COSMETIC AND DERMATOLOGIC FIELDS

(30) Priorité: 03.12.1998 FR 9815293
(43) Date de publication de la demande: 07.06.2000
(73) Titulaire: L'OREAL, 75008 Paris (FR)
(72) Inventeur: Roulier, Véronique, 75010 Paris (FR); Simon, Pascal, 94400 Vitry/S/Seine (FR)
(74) Mandataire: Rasson, Catherine

(56) Documents cités:
- US-A- 4 536 519
- PATENT ABSTRACTS OF JAPAN vol. 011, no. 133 (C-418), 25 avril 1987 (1987-04-25) & JP 61 271029 A (KANEBO LTD), 1 décembre 1986 (1986-12-01)

## Description

La présente invention se rapporte à une composition sous forme d'une émulsion huile-dans-eau (H/E) comportant une forte teneur en cire, et à ses utilisations dans les domaines cosmétique et dermatologique, notamment pour le soin, le traitement et/ou le maquillage de la peau et/ou des muqueuses, et plus particulièrement pour le traitement des rides et/ou ridules de la peau et/ou pour le traitement et/ou la protection de la peau sèche.

L'invention concerne également un procédé de préparation de cette composition selon lequel on effectue au moins une étape du procédé à l'aide d'un mélangeur-extrudeur.

Il est connu d'utiliser des cires dans des crèmes cosmétiques se présentant sous forme d'émulsions et destinées au soin de la peau humaine, notamment pour les effets antirides apportés par ces cires. Néanmoins, il est difficile d'incorporer dans ces compositions un fort pourcentage de cires car les cires ont tendance à épaissir énormément les émulsions. En outre, quand on incorpore un fort pourcentage de cires dans une émulsion, celle-ci s'applique très difficilement sur la peau car elle ne glisse pas. De plus, il apparaît un effet rêche sur la peau. L'utilisation d'une telle émulsion est donc rédhibitoire pour l'utilisateur.

De plus, il est connu d'incorporer un fort pourcentage de cires dans des mascaras. Toutefois, ce type de compositions n'est pas utilisable comme produit de soin du fait des inconvénients précédemment cités.

Par ailleurs, pour préparer une émulsion contenant des cires, il est nécessaire de faire fondre les cires dans la phase grasse de l'émulsion, notamment si l'on veut par exemple utiliser des cires telles que les cires de camauba qui sont particulièrement intéressantes pour leur effet antirides sur la peau. Il faut donc chauffer la phase grasse jusqu'à 80-85°C, ce qui est particulièrement préjudiciable lorsque l'on souhaite introduire des composés thermosensibles.

Il subsiste donc le besoin d'une composition ayant la consistance d'une crème et contenant un pourcentage important de cires sans les inconvénients de l'art antérieur.

La demanderesse a trouvé de manière surprenante que l'on pouvait incorporer un fort pourcentage de cires dans des émulsions H/E tout en conservant une fluidité satisfaisante et une sensation agréable lors de l'application sur la peau, en préparant l'émulsion à froid à l'aide d'un émulsionnant anionique liquide à la température ambiante et à partir d'une phase huileuse souple contenant un fort pourcentage de cires.

Les documents US-A-4,536,519 et JP-A-61-271029 décrivent des émulsions H/E contenant un ester d'acide phosphorique, la phase huileuse étant mélangée à la phase aqueuse à chaud, selon le procédé habituellement utilisé.

La présente invention a donc pour objet une composition sous forme d'une crème constituée d'une émulsion huile-dans-eau comportant une phase huileuse dispersée dans une phase aqueuse, caractérisée en ce qu'elle contient au moins un émulsionnant anionique liquide à température ambiante et au moins 5 % en poids d'une ou plusieurs cires par rapport au poids total de la composition, en ce que la phase huileuse est sous la forme d'une pâte souple à la température ambiante, susceptible d'être obtenue en malaxant le mélange obtenu à partir du prémélange fondu des cires et huiles et des autres constituants de la phase huileuse, tout en le refroidissant jusqu'à température ambiante, et en ce que le mélange des phases huileuse et aqueuse se fait à froid.

La composition de l'invention est sous forme d'une crème, c'est-à-dire un produit souple par opposition à un produit solide tel qu'un stick. Une crème a une viscosité à la température ambiante (environ 20-25°C) allant d'environ 1 à 25 Pa.s et de préférence d'environ 1 à 10 Pa.s, cette viscosité étant mesurée avec un Rhéomat 180.

La composition obtenue bien que contenant un fort taux de cire est fraîche à l'application.

L'émulsionnant anionique utilisé dans la composition de l'invention est liquide à la température ambiante, c'est-à-dire à une température allant de 15°C à 25°C. Il peut être choisi notamment dans le groupe des tensioactifs anioniques à groupement phosphate, liquides à la température ambiante, tels que les mono, di- et/ou tri-esters d'acide phosphorique et d'alcool gras en C12 à C22, c'est-à-dire ayant une chaîne alkyle comportant de 12. à 22 atomes de carbone, et les mono, di- et/ou tri-esters d'acide phosphorique et d'alcool gras éthoxylé, ayant une chaîne grasse comportant de 12 à 22 atomes de carbone et un nombre de motifs éthoxylés allant de 1 à 100 et de préférence de 4 à 25.

On peut citer notamment comme émulsionnant anionique liquide à la température ambiante, le phosphate d'oléyle (mélange de mono- et de diester d'acide phosphorique et d'alcool oléique), le phosphate de trioléyle, le phosphate de dilaureth-4 (mélange de diesters d'acide phosphorique et d'éther de polyéthylène glycol et d'alcool laurique, ayant 4 groupes oxyéthylénés), le phosphate detrioleth-8 (mélange de tresters d'acide phosphorique et d'éther de polyéthylène glycol et d'alcool oléique, ayant 8 groupes oxyéthylénés), le phosphate de triceteareth-4 (mélange de triesters d'acide phosphorique et d'éther de polyéthylène glycol et d'alcools cétylique et stéarylique, ayant 4 groupes oxyéthylénés), le mono-ester d'acide phosphorique et d'acides stéarique et isostéarique (nom CTFA : octyldecyl phosphate) vendu sous la dénomination Hostaphat CG 120 par la société Clariant, et leurs mélanges.

Selon un mode préféré de réalisation de l'invention, on utilise l'octyldecyl phosphate.

La quantité d'émulsionnant dans la composition selon l'invention va généralement de 0,1 à 10 % en poids en matière active et de préférence de 1 à 5 % en poids en matière active par rapport au poids total de la composition.

On peut éventuellement ajouter à l'émulsionnant anionique liquide à la température ambiante, un co-émulsionnant du moment que le mélange de l'émulsionnant et du co-émulsionnant est liquide à température ambiante.

Comme co-émulsionnant, on peut citer par exemple les alcools gras comportant une chaîne ramifiée ou insaturée ayant de 8 à 22 atomes de carbone, tels que l'alcool isostéarique, les acides gras comportant une chaîne ramifiée ou insaturée ayant de 8 à 22 atomes de carbone, tels que l'acide ricinoléique, et leurs mélanges.

Quand la composition contient un co-émulsionnant, la quantité de co-émulsionnant va généralement de 0,1 à 10 % en poids en matière active et de préférence de 1 à 5 % en poids en matière active par rapport au poids total de la composition.

La composition de l'invention contient dans la phase huileuse, au moins 5 % en poids d'une ou plusieurs cires, par rapport au poids total de la composition. La phase huileuse refroidie avant son mélange avec la phase aqueuse se présente de façon avantageuse sous forme d'une pâte souple à la température ambiante (environ 25°C). On entend ici par "pâte souple" une pâte dont on peut mesurer la viscosité, par opposition à la structure solide d'un bâton ou stick, dont on ne peut pas mesurer la viscosité. La viscosité dynamique de la pâte souple à 25°C est généralement comprise entre 3 et 35 Pa.s, mesurée avec un viscosimètre rotatif CONTRAVES TV équipé d'un mobile "MS-r4" à la fréquence de 60 Hz.

Le fait que la phase huileuse soit sous forme d'une pâte souple à la température ambiante permet de préparer l'émulsion à froid et d'obtenir une composition crémeuse facile à étaler sur la peau et agréable à utiliser, et ce malgré le taux important de cires.

Comme cires utilisables dans la composition de l'invention, on peut citer par exemple les cires minérales telles que les cires microcristallines, la paraffine, le petrolatum, la vaseline, l'ozokérite, la cire de montan ; les cires animales telles que la cire d'abeilles, la lanoline et ses dérivés ; les cires végétales telles que les cires de Candellila, d'Ourrury, de Carnauba, du Japon, le beurre de cacao, les cires de fibres de lièges ou de canne à sucre ; les huiles hydrogénées concrètes à 25°C ; les esters gras et les glycérides concrets à 25°C ; les cires synthétiques telles que les cires de polyéthylène et les cires obtenues par synthèse de Fischer-Tropsch ; les cires de silicone, et leurs mélanges.

Selon un mode préféré de réalisation de l'invention, on utilise au moins une cire ayant une température de fusion commençante supérieure ou égale à 50°C, et mieux au moins une cire dont la température de fusion commençante est supérieure à 65°C, telles que la cire de Carnauba, certaines cires de polyéthylène et certaines cires microcristallines telles que celle vendue par la société Tisco sous le nom "Tisco Wax 88" ou celle vendue par la société RMC sous le nom de "Feruwax 30540".

Par "température de fusion commençante", on entend dans la présente description la température à laquelle une cire commence à fondre. On peut déterminer cette température par ATD (analyse thermique différentielle) qui permet l'obtention du thermogramme (ou courbe de fusion) de la cire considérée. La température de fusion commençante correspond à la température à laquelle on peut observer un changement de pente notable dans le thermogramme. Le point de fusion, quant à lui, représente le point minimum dudit thermogramme.

La quantité de cire(s) dans la composition de l'invention est d'au moins 5 % et va de préférence de 5 à 30 % et mieux de 5 à 15 % en poids par rapport au poids total de la composition.

La quantité de phase huileuse contenant au moins une cire, dans la composition de l'invention va généralement de 10 à 70 % et de préférence de 20 à 50 % en poids par rapport au poids total de la composition. Cette phase huileuse est utilisée en une quantité telle ou bien contient une quantité de cires telle que la quantité de cires dans la composition finale est égale ou supérieure à 5 %.

La phase huileuse de la composition de l'invention comprend généralement, outre la ou les cires, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone tels que les produits commercialisés sous la dénomination "KSG" par la société Shin-Etsu, sous la dénomination "Trefil" par la société Dow Corning ou sous la dénomination "Gransil" par la société Général Electric.

De manière avantageuse, la composition de l'invention peut aussi contenir une ou plusieurs charges (constituants pulvérulents) qui peuvent être choisies par exemple dans le groupe formé par le talc ; les micas d'origine naturelle ou synthétique ; le kaolin ; les oxydes de zinc ou de titane ; le carbonate de calcium ; le carbonate et l'hydrocarbonate de magnésium ; la silice, en particulier la silice sphérique, la poudre de silice commercialisée sous la dénomination "Cab-O-Sil TS 530" par la société Cabot, et les microbilles de silice telles que celles commercialisées sous la dénomination SB150 par la société Myoshi ; le dioxyde de titane ; les billes de verre et de céramique commercialisées par la société 3M sous la dénomination commerciale "Macrolite" ; les savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium ; les poudres de polymères synthétiques non expansées, telles que les poudres de polyéthylène, de polystyrène, de polyesters, de polyamides (par exemple le nylon ou la poly-β-alanine), de copolymères d'acrylates (par exemple les microsphères microporeuses vendues par la société Dow Corning sous la dénomination commerciale "Polytrap"), d'acides polyméthacryliques, de polystyrène, de téflon comme le "Fluon"; les poudres expansées telles que les microsphères creuses en matériau thermoplastique préparées par des procédés connus, comme ceux décrits dans US-A-3 615 972 et EP-A-0 56219 et notamment les microsphères commercialisées sous la dénomination commerciale "Expancel" par la société Kemanord Plast ou sous la dénomination commerciale "Micropearl F 80 ED" par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination "Dry-Flo" par la société National Starch ; les microbilles de résine de silicone telles que celles commercialisées sous la dénomination "Tospearl" par la société Toshiba Silicone, et leurs mélanges.

Les charges peuvent représenter jusqu'à 20 % en poids par rapport au poids total de la composition, et de préférence de 1 à 12 % en poids par rapport au poids total de la composition.

La phase aqueuse de la composition de l'invention représente au moins 30 % en poids et de préférence de 50 à 80 % en poids par rapport au poids total de la composition.

La composition selon l'invention peut être utilisée dans tous les domaines où ce type de forme galénique est intéressant, et notamment dans les domaines cosmétique et dermatologique. Quand elle constitue une composition cosmétique et/ou dermatologique, elle contient avantageusement un milieu physiologiquement acceptable, c'est-à-dire compatible avec la peau, les muqueuses, les ongles et/ou les cheveux.

Les compositions, objet de l'invention, trouvent leur application dans un grand nombre de traitements de la peau, des muqueuses (lèvres) et des cheveux, y compris le cuir chevelu, notamment pour la protection, le soin, le nettoyage et/ou le maquillage de la peau et/ou des muqueuses, pour la protection, le soin et/ou le nettoyage des cheveux et/ou pour le traitement de la peau, des cheveux et/ou des muqueuses.

Les compositions selon l'invention peuvent par exemple être utilisées comme produits de traitement, de soin, de protection et/ou de nettoyage de la peau sous forme de crèmes ou de laits, ou comme produits de maquillage (peau et lèvres) par incorporation de charges et/ou de matières colorantes (pigments et/ou colorants). Elles sont particulièrement appropriées pour le traitement des rides et/ou ridules de la peau et pour le traitement et/ou la protection de la peau sèche.

Aussi, l'invention a encore pour objet l'utilisation cosmétique de la composition telle que définie ci-dessus pour le traitement, la protection, le soin et/ou le nettoyage de la peau, des muqueuses et/ou les cheveux, et/ou pour le maquillage de la peau et/ou des muqueuses.

Elle a aussi pour objet l'utilisation cosmétique de la composition telle que définie ci-dessus pour le traitement des rides et/ou des ridules de la peau.

L'invention a encore pour objet l'utilisation de la composition telle que définie ci-dessus pour la fabrication d'une composition destinée au traitement et/ou à la protection de la peau sèche.

En outre, de façon connue, les compositions de l'invention peuvent contenir des adjuvants habituels dans le domaine cosmétique ou dermatologique, tels que des actifs hydrophiles ou lipophiles, des conservateurs, des antioxydants, des parfums, des solvants, des filtres solaires, des matières colorantes, des agents basiques ou acides et encore des vésicules lipidiques. Ces adjuvants sont utilisés dans les proportions habituelles dans le domaine cosmétique ou dermatologique, et par exemple de 0,01 à 30 % du poids total de la composition, et ils sont, selon leur nature, introduits dans la phase aqueuse ou dans la phase huileuse de la composition, ou encore dans des vésicules. Ces adjuvants ainsi que leurs concentrations doivent être tels qu'ils ne modifient pas la propriété recherchée pour la composition.

Si l'on souhaite obtenir une composition moins fluide et/ou améliorer la stabilité de l'émulsion, on peut y ajouter un ou plusieurs gélifiants hydrophiles tels que les polymères carboxyvinyliques ou carbomers et les polyacrylamides. Ces gélifiants sont utilisés à des concentrations allant généralement de 0,05 à 2 %, de préférence 0,1 à 0,5 % en poids par rapport au poids total de la composition.

Comme actifs utilisables dans la composition de l'invention, on peut citer par exemple les hydratants tels que les polyols et notamment la glycérine, l'éthylène glycol, l'isoprène-glycol, le propane-1,2 diol, la diglycérine, le sorbitol, les polyéthylène glycols et leurs mélanges.

La composition selon l'invention peut être préparée de manière avantageuse en utilisant, pour au moins une étape du procédé, un appareil de malaxage tel qu'un broyeur à cylindres comportant deux cylindres tournant en sens inverse entre lesquels passe la pâte ou un mélangeur-extrudeur à vis. On utilise de préférence un mélangeur-extrudeur à vis.

Un autre objet de l'invention est donc un procédé de préparation d'une composition selon l'invention, caractérisé en ce que l'on effectue au moins une étape du procédé à l'aide d'un mélangeur-extrudeur à vis.

Selon un premier mode de réalisation de l'invention, le procédé de préparation comprend les étapes suivantes :
- (1) préparation de la phase huileuse sous forme d'une pâte souple obtenue en faisant un prémélange des cires et huiles, en chauffant ce prémélange à une température à laquelle il fond, puis en introduisant dans un mélangeur-extrudeur à vis soumis à un gradient de température allant de 80°C à 20°C, en une ou plusieurs fois, le prémélange fondu et les autres constituants (notamment les charges) de la phase huileuse, et en malaxant le mélange obtenu tout en le refroidissant jusqu'à température ambiante tandis qu'il est amené à la sortie du mélangeur-extrudeur ;
- (2) incorporation de l'émulsionnant et éventuellement du co-émulsionnant dans la pâte souple obtenue en (1), et
- (3) incorporation, sous agitation, du mélange obtenu en (2) dans la phase aqueuse.

Dans ce mode de réalisation, les étapes (2) et (3) sont réalisées dans un appareil de mélange habituellement utilisé par l'homme du métier, tel qu'un rotor stator.

En outre, dans le procédé décrit ci-dessus, l'émulsionnant utilisé est un émulsionnant anionique liquide à température ambiante et les quantités utilisées sont telles que l'émulsion obtenue comporte au moins 5 % de cire en poids par rapport au poids total de la composition.

Comme indiqué plus haut, le mélange des phase huileuse et aqueuse se faisant à froid, l'incorporation de composés thermosensibles ne pose pas de problème.

Selon un mode particulier de réalisation de l'invention, les étapes (2) et (3) ci-dessus sont également réalisées dans le mélangeur-extrudeur à vis utilisé pour l'étape (1). L'émulsionnant et la phase aqueuse sont alors introduits dans une partie (ou élément) du mélangeur-extrudeur où la température est proche de la température ambiante.

L'utilisation d'un mélangeur-extrudeur permet d'obtenir de façon reproductible une pâte de phase huileuse de qualité très constante. De plus, il est possible, en adaptant la filière de sortie du mélangeur-extrudeur, de conditionner la composition en ligne à la sortie dudit mélangeur-extrudeur.

Les différents étapes du procédé peuvent être réalisées dans un ou plusieurs extrudeurs disposés à la suite les uns des autres, et de préférence dans un extrudeur bivis unique.

Les conditions dans lesquelles l'extrusion peut être effectuée sont décrites dans le document FR-A-2,715,306 dont le contenu est incorporé à la présente demande par référence.

L'invention est illustrée plus en détail dans l'exemple suivant. Les quantités indiquées sont des pourcentages en poids.

### Exemple : crème de soin

### Phase huileuse

- Dry-Flo (charge) 15 %
- Cire microcristalline 19 %
- Huile minérale qsp 100 %

### Emulsion H/E

- Phase huileuse 20 %
- Hostaphat CG 120 (émulsionnant) 3 %
- Carbomer 0,2 %
- Eau qsp 100 %

### Mode opératoire 1 :

- On chauffe le mélange de cire et d'huile à environ 100°C,
- on introduit le mélange fondu dans un mélangeur-extrudeur en même temps que la charge, on obtient à la sortie du mélangeur-extrudeur la phase huileuse sous forme d'une pâte souple,
- Dans un rotor stator, on incorpore dans la pâte souple l'émulsionnant,
- puis on ajoute peu à peu le mélange obtenu dans la phase aqueuse (eau et carbomer) tout en agitant.

### Mode opératoire 2:

- On chauffe le mélange de cire et d'huile à environ 100°C,
- on introduit la charge dans l'élément de tête d'un mélangeur-extrudeur comportant au moins six éléments,
- on introduit la phase huileuse dans le second élément dudit mélangeur-extrudeur à vis, et
- on introduit la phase aqueuse et l'émulsionnant par deux entrées différentes, dans le quatrième élément dudit mélangeur-extrudeur à vis.

Les éléments du mélangeur-extrudeur à vis utilisé sont, en allant du premier au sixième élément, portés respectivement aux températures suivantes : 20°C, 80°C, 60°C, 20°C, 20°C et 20°C.

On obtient une crème qui présente une texture très légère et qui possède de bonnes qualités d'hydratation et est apte à lisser le relief de la peau.

## Revendications

1. Composition sous forme d'une crème constituée d'une émulsion huile-dans-eau comportant une phase huileuse dispersée dans une phase aqueuse, **caractérisée en ce qu'**elle contient au moins un émulsionnant anionique liquide à température ambiante et au moins 5 % en poids d'une ou plusieurs cires par rapport au poids total de la composition, **en ce que** la phase huileuse est sous la forme d'une pâte souple à la température ambiante, susceptible d'être obtenue en malaxant le mélange obtenu à partir du prémélange fondu des cires et huiles et des autres constituants de la phase huileuse, tout en le refroidissant jusqu'à température ambiante, et **en ce que** le mélange des phases huileuse et aqueuse se fait à froid.

2. Composition selon la revendication 1, **caractérisée en ce que** l'émulsionnant anionique liquide à température ambiante est choisi dans le groupe comprenant les tensioactifs anioniques à groupement phosphate.

3. Composition selon la revendication 1 ou 2, **caractérisée en ce que** l'émulsionnant liquide à température ambiante est choisi dans le groupe comprenant les mono, di- et/ou tri-esters d'acide phosphorique et d'alcool gras ayant une chaîne grasse comportant de 12 à 22 atomes de carbone et les mono, di- et/ou tri-esters d'acide phosphorique et d'alcool gras éthoxylé, ayant une chaîne grasse comportant de 12 à 22 atomes de carbone et un nombre de motifs éthoxylés allant de 1 à 100.

4. Composition selon l'une quelconque des revendications précédentes, **caractérisée en ce que** l'émulsionnant liquide à température ambiante est choisi dans le groupe comprenant le phosphate d'oléyle, le phosphate de trioléyle, le phosphate de dilaureth-4, le phosphate de trioleth-8, le phosphate de triceteareth-4, le mono-ester d'acide phosphorique et d'acides stéarique et isostéarique, et leurs mélanges.

5. Composition selon l'une quelconque des revendications précédentes, **caractérisée en ce que** la quantité d'émulsionnant va de 0,1 à 10 % en poids de matière active par rapport au poids total de la composition.

6. Composition selon l'une quelconque des revendications précédentes, **caractérisée en ce que** la composition contient en outre un co-émulsionnant.

7. Composition selon la revendication précédente, **caractérisée en ce que** le co-émulsionnant est choisi dans le groupe comprenant les alcools gras comportant une chaîne ramifiée ou insaturée ayant de 8 à 22 atomes de carbone, les acides gras comportant une chaîne ramifiée ou insaturée ayant de 8 à 22 atomes de carbone et leurs mélanges.

8. Composition selon la revendication 6 ou 7, **caractérisée en ce que** la quantité de co-émulsionnant va de 0,1 à 10 % en poids de matière active par rapport au poids total de la composition.

9. Composition selon l'une quelconque des revendications précédentes, **caractérisée en ce que** la cire est choisie dans le groupe comprenant les cires minérales, les cires animales, les cires végétales, les huiles hydrogénées concrètes à 25°C, les esters gras et les glycérides concrets à 25°C, les cires synthétiques, les cires de silicone et leurs mélanges.

10. Composition selon l'une quelconque des revendications précédentes, **caractérisée en ce qu'**elle contient au moins une cire choisie parmi les cires ayant une température de fusion commençante supérieure ou égale à 50°C.

11. Composition selon l'une quelconque des revendications précédentes, **caractérisée en ce que** la cire est choisie parmi la cire de Carnauba, les cires de polyéthylène ayant une température de fusion commençante supérieure à 65°C, les cires microcristallines ayant une température de fusion commençante supérieure à 65°C, et leurs mélanges.

12. Composition selon l'une quelconque des revendications précédentes, **caractérisée en ce que** la quantité de cire(s) va de 5 à 30 % en poids par rapport au poids total de la composition.

13. Composition selon l'une quelconque des revendications précédentes, **caractérisée en ce que** la quantité de phase huileuse va de 10 à 70 % en poids par rapport au poids total de la composition.

14. Composition selon l'une quelconque des revendications précédentes, **caractérisée en ce que** la phase huileuse comprend, en outre, un ou plusieurs corps gras choisis parmi les huiles d'origine animale, les huiles d'origine végétale, les huiles minérales, les huiles synthétiques, les huiles fluorées, les huiles de silicone et notamment les huiles de silicone volatiles, les gommes de silicone, les résines de silicone, les alcools gras, les acides gras et les élastomères de silicone.

15. Composition selon l'une quelconque des revendications précédentes, **caractérisée en ce qu'**elle comprend en outre au moins une charge.

16. Composition selon la revendication précédente, **caractérisée en ce que** la quantité de charge(s) va de 1 à 12 % en poids par rapport au poids total de la composition.

17. Composition selon l'une quelconque des revendications précédentes, **caractérisée en ce que** la phase aqueuse représente de 50 à 80 % en poids par rapport au poids total de la composition.

18. Composition selon l'une quelconque des revendications précédentes, **caractérisée en ce qu'**elle constitue une composition cosmétique et/ou dermatologique.

19. Utilisation cosmétique de la composition selon l'une quelconque des revendications précédentes pour le traitement, la protection, le soin et/ou le nettoyage de la peau, des muqueuses et/ou les cheveux, et/ou pour le maquillage de la peau et/ou des muqueuses.

20. Utilisation cosmétique de la composition selon l'une quelconque des revendications 1 à 18 pour le traitement des rides et/ou des ridules de la peau.

21. Utilisation de la composition selon l'une quelconque des revendications 1 à 18 pour la fabrication d'une composition destinée au traitement et/ou à la protection de la peau sèche.

22. Procédé de préparation d'une composition selon l'une quelconque des revendications 1 à 18, **caractérisé en ce que** l'on effectue au moins une étape du procédé à l'aide d'un mélangeur-extrudeur à vis.

23. Procédé selon la revendication 22, **caractérisé en ce qu'**il comprend les étapes suivantes :
- (1) préparation de la phase huileuse sous forme d'une pâte souple obtenue en faisant un prémélange des cires et huiles, en chauffant ce prémélange à une température à laquelle il fond, puis en introduisant dans un mélangeur-extrudeur à vis soumis à un gradient de température allant de 80°C à 20°C, en une ou plusieurs fois, le prémélange fondu et les autres constituants de la phase huileuse, et en malaxant le mélange obtenu tout en le refroidissant jusqu'à température ambiante tandis qu'il est amené à la sortie du mélangeur-extrudeur ;
- (2) incorporation de l'émulsionnant et éventuellement le co-émulsionnant dans la pâte souple obtenue en (1), et
- (3) incorporation, sous agitation, du mélange obtenu en (2) dans la phase aqueuse.

24. Procédé selon la revendication 22 ou 23, **caractérisé en ce que** les étapes (2) et (3) sont réalisées dans le mélangeur-extrudeur à vis de l'étape (1).

25. Procédé selon l'une quelconque des revendications 22 à 24, **caractérisé en ce que** la phase huileuse se présente sous forme d'une pâte souple ayant une viscosité dynamique à 25°C comprise entre 3 et 35 Pa.s, mesurée avec un viscosimètre rotatif CONTRAVES TV équipé d'un mobile "MS-r4" à la fréquence de 60 Hz.

## Patentansprüche

1. Zusammensetzung in Form einer Creme, die aus einer Öl-in-Wasser-Emulsion besteht, die eine in einer wässerigen Phase dispergierte Ölphase aufweist, **dadurch gekennzeichnet, dass** sie mindestens einen bei Umgebungstemperatur flüssigen anionischen Emulgator und mindestens 5 Gew.-% eines Wachses oder mehrerer Wachse, bezogen auf das Gesamtgewicht der Zusammensetzung, enthält, dadurch, dass die Ölphase bei Umgebungstemperatur in Form einer weichen Paste vorliegt, die durch Kneten des Gemisches, das aus dem geschmolzenen Vorgemisch der Wachse und Öle und der weiteren Bestandteile der Ölphase erhalten wird, hergestellt werden kann, wobei es auf Umgebungstemperatur abgekühlt wird, und dadurch, dass das Mischen der Ölphase mit der wässerigen Phase in der Kälte erfolgt.

2. Zusammensetzung nach Anspruch 1, **dadurch gekennzeichnet, dass** der bei Umgebungstemperatur flüssige anionische Emulgator unter anionischen grenzflächenaktiven Stoffen mit Phosphatgruppe ausgewählt ist.

3. Zusammensetzung nach Anspruch 1 oder 2, **dadurch gekennzeichnet, dass** der bei Umgebungstemperatur flüssige Emulgator unter Mono-, Di- und/oder Triestern von Phosphorsäure und einem Fettalkohol, der eine Fettkette mit 12 bis 22 Kohlenstoffatomen aufweist, und Mono-, Di- und/oder Triestern von Phosphorsäure und einem ethoxyliertem Fettalkohol, der eine Fettkette mit 12 bis 22 Kohlenstoffatomen aufweist und bei dem die Anzahl ethoxylierter Einheiten im Bereich von 1 bis 100 liegt, ausgewählt ist.

4. Zusammensetzung nach einem der vorhergehenden Ansprüche, **dadurch gekennzeichnet, dass** der bei Umgebungstemperatur flüssige Emulgator unter Oleylphosphat, Trioleylphosphat, Dilaureth-4-phosphat, Trioleth-8-phosphat, Triceteareth-4-phosphat, dem Monoester von Phosphorsäure und Stearin- und Isostearinsäure und den Gemischen dieser Verbindungen ausgewählt ist.

5. Zusammensetzung nach einem der vorhergehenden Ansprüche, **dadurch gekennzeichnet, dass** der Mengenanteil des Emulgators im Bereich von 0,1 bis 10 Gew.-% (Wirkstoff), bezogen auf das Gesamtgewicht der Zusammensetzung, liegt.

6. Zusammensetzung nach einem der vorhergehenden Ansprüche, **dadurch gekennzeichnet, dass** die Zusammensetzung ferner einen Coemulgator enthält.

7. Zusammensetzung nach dem vorhergehenden Anspruch, **dadurch gekennzeichnet, dass** der Coemulgator unter Fettalkoholen, die eine verzweigte oder ungesättigte Kette mit 8 bis 22 Kohlenstoffatomen aufweisen, Fettsäuren, die eine verzweigte oder ungesättigte Kette mit 8 bis 22 Kohlenstoffatomen aufweisen, und den Gemischen dieser Verbindungen ausgewählt ist.

8. Zusammensetzung nach Anspruch 6 oder 7, **dadurch gekennzeichnet, dass** der Mengenanteil des Coemulgators im Bereich von 0,1 bis 10 Gew.-% (Wirkstoff), bezogen auf das Gesamtgewicht der Zusammensetzung, liegt.

9. Zusammensetzung nach einem der vorhergehenden Ansprüche, **dadurch gekennzeichnet, dass** das Wachs unter mineralischen Wachsen, tierischen Wachsen, pflanzlichen Wachsen, bei 25 °C festen hydrierten Ölen, bei 25 °C festen Fettestern und Glyceriden, synthetischen Wachsen, Siliconwachsen und den Gemischen dieser Verbindungen ausgewählt ist.

10. Zusammensetzung nach einem der vorhergehenden Ansprüche, **dadurch gekennzeichnet, dass** sie mindestens ein Wachs enthält, das unter den Wachsen ausgewählt ist, deren Startschmelztemperatur größer oder gleich 50 °C ist.

11. Zusammensetzung nach einem der vorhergehenden Ansprüche, **dadurch gekennzeichnet, dass** das Wachs unter Carnaubawachs, Polyethylenwachsen mit einer Startschmelztemperatur größer 65 °C, mikrokristallinen Wachsen mit einer Startschmelztemperatur größer 65 °C und den Gemischen dieser Verbindungen ausgewählt ist.

12. Zusammensetzung nach einem der vorhergehenden Ansprüche, **dadurch gekennzeichnet, dass** der Mengenanteil des Wachses (der Wachse) im Bereich von 5 bis 30 Gew.-%, bezogen auf das Gesamtgewicht der Zusammensetzung, liegt.

13. Zusammensetzung nach einem der vorhergehenden Ansprüche, **dadurch gekennzeichnet, dass** der Mengenanteil der Ölphase im Bereich von 10 bis 70 Gew.-%, bezogen auf das Gesamtgewicht der Zusammensetzung, liegt.

14. Zusammensetzung nach einem der vorhergehenden Ansprüche, **dadurch gekennzeichnet, dass** die Ölphase ferner eine oder mehrere Fettsubstanzen enthält, die unter Ölen tierischen Ursprungs, Ölen pflanzlichen Ursprungs, Mineralölen, synthetischen Ölen, fluorierten Ölen, Siliconölen und insbesondere flüchtigen Siliconölen, Silicongummis, Siliconharzen, Fettalkoholen, Fettsäuren und Siliconelastomeren ausgewählt sind.

15. Zusammensetzung nach einem der vorhergehenden Ansprüche, **dadurch gekennzeichnet, dass** sie ferner mindestens einen Füllstoff enthält.

16. Zusammensetzung nach dem vorhergehenden Anspruch, **dadurch gekennzeichnet, dass** der Mengenanteil des Füllstoffs (der Füllstoffe) im Bereich von 1 bis 12 Gew.-%, bezogen auf das Gesamtgewicht der Zusammensetzung, liegt.

17. Zusammensetzung nach einem der vorhergehenden Ansprüche, **dadurch gekennzeichnet, dass** die wässerige Phase 50 bis 80 Gew.-%, bezogen auf das Gesamtgewicht der Zusammensetzung, ausmacht.

18. Zusammensetzung nach einem der vorhergehenden Ansprüche, **dadurch gekennzeichnet, dass** sie eine kosmetische und/oder dermatologische Zusammensetzung darstellt.

19. Kosmetische Verwendung der Zusammensetzung nach einem der vorhergehenden Ansprüche zur Behandlung, zum Schutz, zur Pflege und/oder zur Reinigung der Haut, der Schleimhäute und/oder der Haare und/oder zum Schminken der Haut und/oder der Schleimhäute.

20. Kosmetische Verwendung der Zusammensetzung nach einem der Ansprüche 1 bis 18 zur Behandlung von Hautfalten und/oder Hautfältchen.

21. Verwendung der Zusammensetzung nach einem der Ansprüche 1 bis 18 zur Herstellung einer Zusammensetzung, die zur Behandlung und/oder zum Schutz von trockener Haut bestimmt ist.

22. Verfahren zur Herstellung einer Zusammensetzung nach einem der Ansprüche 1 bis 18, **dadurch gekennzeichnet, dass** mindestens ein Schritt des Verfahrens mit einem Mischextruder mit Schnecke durchgeführt wird.

23. Verfahren nach Anspruch 22, **dadurch gekennzeichnet, dass** es die folgenden Schritte umfasst:
(1) Herstellung der Ölphase in Form einer weichen Paste, die dadurch erhalten wird, dass ein Vorgemisch der Wachse und Öle hergestellt wird, dieses Vorgemisch auf eine Temperatur erwärmt wird, bei der es schmilzt, anschließend das geschmolzene Vorgemisch und die weiteren Bestandteile der Ölphase auf einmal oder auf mehrere Male in einen Mischextruder mit Schnecke eingebracht werden, in dem ein Temperaturgradient von 80 bis 20 °C vorliegt, und dann das erhaltene Gemisch geknetet wird, wobei auf Umgebungstemperatur abgekühlt wird, während es zum Ausgang des Mischextruders gebracht wird;
(2) Einarbeiten des Emulgators und gegebenenfalls des Coemulgators in die bei (1) erhaltene weiche Paste und
(3) Einarbeiten des bei (2) erhaltenen Gemisches unter Rühren in die wässerige Phase.

24. Verfahren nach Anspruch 22 oder 23, **dadurch gekennzeichnet, dass** die Schritte (2) und (3) in dem Mischextruder mit Schnecke von Schritt (1) durchgeführt werden.

25. Verfahren nach einem der Ansprüche 22 bis 24, **dadurch gekennzeichnet, dass** die Ölphase in Form einer weichen Paste vorliegt, deren dynamische Viskosität, bestimmt mit einem Rotationsviskosimeter CONTRAVES TV, das mit einem mobilen Teil "MS-r4" mit der Frequenz von 60 Hz ausgestattet ist, bei 25 °C im Bereich von 3 bis 35 Pa·s liegt.

## Claims

1. Composition in the form of a cream comprising an oil-in-water emulsion comprising an oily phase dispersed in an aqueous phase, **characterized in that** it contains at least one anionic emulsifier which is liquid at room temperature and at least 5% by weight of one or more waxes relative to the total weight of the composition, **in that** the oily phase is in the form of a soft paste at room temperature, which may be obtained by blending the mixture obtained from the molten premix of the waxes and oils and the other constituents of the oily phase, while at the same time cooling to room temperature, and **in that** the oily and aqueous phase are mixed together when cold.

2. Composition according to Claim 1, **characterized in that** the anionic emulsifier which is liquid at room temperature is chosen from the group comprising anionic surfactants containing a phosphate group.

3. Composition according to Claim 1 or 2, **characterized in that** the emulsifier which is liquid at room temperature is chosen from the group comprising mono-, di- and/or triesters of phosphoric acid and of a fatty alcohol having a fatty chain containing from 12 to 22 carbon atoms, and mono-, di- and/or triesters of phosphoric acid and of an ethoxylated fatty alcohol, having a fatty chain containing from 12 to 22 carbon atoms and a number of ethoxylated units ranging from 1 to 100.

4. Composition according to any one of the preceding claims, **characterized in that** the emulsifier which is liquid at room temperature is chosen from the group comprising oleyl phosphate, trioleyl phosphate, dilaureth-4 phosphate, trioleth-8 phosphate, triceteareth-4 phosphate, the monoester of phosphoric acid and of stearic and isostearic acid, and mixtures thereof.

5. Composition according to any one of the preceding claims, **characterized in that** the amount of emulsifier ranges from 0.1 to 10% by weight of active material relative to the total weight of the composition.

6. Composition according to any one of the preceding claims, **characterized in that** the composition also comprises a co-emulsifier.

7. Composition according to the preceding claim, **characterized in that** the co-emulsifier is chosen from the group comprising fatty alcohols containing a branched or unsaturated chain containing from 8 to 22 carbon atoms, fatty acids containing a branched or unsaturated chain containing from 8 to 22 carbon atoms, and mixtures thereof.

8. Composition according to Claim 6 or 7, **characterized in that** the amount of co-emulsifier ranges from 0.1 to 10% by weight of active material relative to the total weight of the composition.

9. Composition, according to any one of the preceding claims, **characterized in that** the wax is chosen from the group comprising mineral waxes, animal waxes, plant waxes, hydrogenated oils which are solid at 25°C, fatty esters and glycerides which are solid at 25°C, synthetic waxes, silicone waxes and mixtures thereof.

10. Composition according to any one of the preceding claims, **characterized in that** it contains at least one wax chosen from waxes with a starting melting point of greater than or equal to 50°C.

11. Composition according to any one of the preceding claims, **characterized in that** the wax is chosen from carnauba wax, polyethylene waxes with a starting melting point of greater than 65°C, microcrystalline waxes with a starting melting point of greater than 65°C, and mixtures thereof.

12. Composition according to any one of the preceding claims, **characterized in that** the amount of wax(es) ranges from 5 to 30% by weight relative to the total weight of the composition.

13. Composition according to any one of the preceding claims, **characterized in that** the amount of oily phase ranges from 10 to 70% by weight relative to the total weight of the composition.

14. Composition according to any one of the preceding claims, **characterized in that** the oily phase also comprises one or more fatty substances chosen from oils of animal origin, oils of plant origin, mineral oils, synthetic oils, fluoro oils, silicone oils and in particular volatile silicone oils, silicone gums, silicone resins, fatty alcohols, fatty acids and silicone elastomers.

15. Composition according to any one of the preceding claims, **characterized in that** it also comprises at least one filler.

16. Composition according to the preceding claim, **characterized in that** the amount of filler(s) ranges from 1 to 12% by weight relative to the total weight of the composition.

17. Composition according to any one of the preceding claims, **characterized in that** the aqueous phase represents from 50 to 80% by weight relative to the total weight of the composition.

18. Composition according to any one of the preceding claims, **characterized in that** it constitutes a cosmetic and/or dermatological composition.

19. Cosmetic use of the composition according to any one of the preceding claims, for treating, protecting, caring for and/or cleansing the skin, mucous membranes and/or the hair, and/or for making up the skin and/or mucous membranes.

20. Cosmetic use of the composition according to any one of Claims 1 to 18, for treating wrinkles and/or fine lines of the skin.

21. Use of the composition according to any one of Claims 1 to 18 for the manufacture of a composition intended for treating and/or protecting dry skin.

22. Process for preparing a composition according to any one of Claims 1 to 18, **characterized in that** at least one step of the process is carried out using a mixer-screw extruder.

23. Process according to Claim 22, **characterized in that** it comprises the following steps:
- (1) preparation of the oily phase in the form of a soft paste obtained by preparing a premix of the waxes and oils, heating this premix to a temperature at which it melts and then introducing the molten premix and the other constituents of the oily phase into a mixer-screw extruder subjected to a temperature gradient ranging from 80°C to 20°C, in one or more portions, and blending the mixture obtained while at the same time cooling it to room temperature while it is conveyed to the outlet of the mixer-extruder;
- (2) incorporation of the emulsifier and optionally the co-emulsifier into the soft paste obtained in (1), and
- (3) incorporation, with stirring, of the mixture obtained in (2) into the aqueous phase.

24. Process according to Claim 22 or 23, **characterized in that** steps (2) and (3) are carried out in the mixer-screw extruder of step (1).

25. Process according to any one of Claims 22 to 24 **characterized in that** the oily phase is in the form of a soft paste with a dynamic viscosity at 25°C of between 3 and 35 Pa.s, measured with a Contraves TV rotary viscometer fitted with an "MS-r4" rotor at a frequency of 60 Hz.
